(19) Europäisches Patentamt
European Patent Office
Office européen des brevets

(11) Publication number: **0 058 466**
**B1**

# (12) EUROPEAN PATENT SPECIFICATION

(45) Date of publication of patent specification: **18.12.85**

(51) Int. Cl.$^4$: **C 07 D 327/00,**
C 07 C 143/00, C 07 D 295/00

(21) Application number: **82300177.1**

(22) Date of filing: **13.01.82**

## (54) Cyclic perfluoroaliphatic acid anhydrides and amide derivatives thereof.

(30) Priority: **30.01.81 US 229871**
**30.01.81 US 229872**

(43) Date of publication of application:
**25.08.82 Bulletin 82/34**

(45) Publication of the grant of the patent:
**18.12.85 Bulletin 85/51**

(84) Designated Contracting States:
**DE FR GB IT**

(56) References cited:
**FR-A-2 304 605**
**GB-A-1 120 304**
**US-A-3 842 019**

**CHEMICAL ABSTRACTS, vol. 60, 1963, column 13244a-g, COLUMBUS OHIO (US)**
**CHEMICAL ABSTRACTS, vol. 94, no. 13, March 30, 1981, abstract no. 102784s, page 687, COLUMBUS OHIO (US)**

(73) Proprietor: **MINNESOTA MINING AND MANUFACTURING COMPANY**
**3M Center, P.O. Box 33427**
**St. Paul, MN 55133 (US)**

(72) Inventor: **Behr, Fred E.**
**2501 Hudson Road P.O. Box 33427**
**St. Paul Minnesota 55133 (US)**
Inventor: **Koshar, Robert J.**
**2501 Hudson Road P.O. Box 33427**
**St. Paul Minnesota 55133 (US)**

(74) Representative: **Baillie, Iain Cameron et al**
**c/o Ladas & Parry Isartorplatz 5**
**D-8000 München 2 (DE)**

**EP 0 058 466 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technical field

This invention relates to cyclic fluorocarbon anhydrides, amide derivatives thereof, and a process for their synthesis. In another aspect, this invention relates to curable compositions containing cationically-sensitive monomers, such as epoxides, and said cyclic fluorocarbon anhydrides or amide derivatives thereof. In yet another aspect, this invention relates to a process for curing cationically-sensitive monomers, utilizing as catalyst said cyclic fluorocarbon anhydrides or amide derivatives thereof.

Background art

Processes of polymerizing and curing cationically-sensitive monomers such as cyclic ethers (e.g., epoxides), vinyl ethers, and N-vinyl compounds, in the presence of catalysts, specifically Lewis acids, such as boron trifluoride, aluminum chloride, and the like, are well known. However, many of these catalysts are highly corrosive to various substrates such as metals. Other known catalysts for the polymerization and curing of cationically-sensitive monomers are undesirably toxic. Further, many of these catalysts rapidly catalyze the polymerization of the monomers with which they are admixed and cannot be used where a definite or prolonged shelf life and/or pot life is desired or required. Though some of these prior art catalysts can be used in a latent form, e.g., $BF_3 \cdot NH_2C_2H_5$, their latency is affected by moisture and prolonged latency is difficult to achieve; in addition, when these latent catalysts are activated, this gives rise to the aforementioned objectionable corrosiveness. Also, many known catalysts are not effective for polymerization of a broad range of cationically-sensitive monomers, e.g., for polymerization of both epoxides and cyclic siloxanes.

Various linear perfluoroaliphaticsulfonic acid anhydrides of the formula $(RSO_2)_2O$, where R is perfluoroalkyl, are described in U.S. Patent No. 2,732,398. U.K. Patent specification No. 1,120,304 discloses the use of the anhydride of trifluoromethanesulfonic acid as a catalyst for use in the polymerization of various cationically-sensitive monomers.

The utility of linear fluorocarbon sulfonic acid anhydrides (e.g., those derived from monofunctional perfluoroaliphaticsulfonic acids) as perfluoroaliphaticsulfonylation or acylation agents is also konwn. Use of the anhydride $(CF_3SO_2)_2O$ as a trifluoromethanesulfonylation agent for formation of trifluoromethanesulfonamides by reaction with ammonia or amines is disclosed in Chemical Reviews, *77*, 69—92 (1977). T. R. Forbus and J. C. Martin, J. Org. Chem., *44*, 313 (1979) have disclosed the preparation of the mixed anhydride, $CF_3SO_2OC(O)CF_3$, and its use as a trifluoroacetylation reagent for aromatic compounds. In these reactions, however, the above linear anhydrides produce not only the desired trifluoromethanesulfonylation or acylation product but also produce an equivalent amount of trifluoromethanesulfonic acid or salt thereof as byproduct. For example, the reaction of $(CF_3SO_2)_2O$ with ammonia provides equal portions of trifluoromethanesulfonamide and the ammonium salt of trifluoromethanesulfonic acid as a by-product. Such by-product is undesirable because of unfavorable economics in the preparation of the desired product.

Cyclic fluorocarbon acid anhydrides are highly desirable compositions since, in contrast to the above linear anhydrides, reaction of cyclic anhydrides with reagents such as ammonia or amines can produce useful difunctional products by ring-opening reactions without formation of the above-described by-products. Very few such cyclic anhydrides are known, however, because of many factors such as ring instability, or decomposition, e.g., decarboxylation, during the process of ring formation, or because of the inability of many difunctional acids to undergo ring closure by dehydration. Cyclic anhydrides such as perfluorosuccinic acid anhydride are well known and provide useful products by ring-opening reactions such as reaction with ammonia to produce ammonium salts of the perfluorocarboxylic acids containing terminal carboxyamide ($CONH_2$) functional groups. However, such cyclic anhydrides or their amide derivative(s) do not exhibit the catalytic properties of the cyclic anhydrides or amide derivatives of the invention described below.

The use of ammonia or amine salts of monofunctional perfluoroaliphaticsulfonic acids as latent catalysts for the polymerization of cationically-sensitive monomers is well known, see U.S. Patent No. 3,842,019 and R. R. Alm, Modern Paint and Coatings, October, 1980, pages 88—92. However, the salts described in these references do not include a second functional group in the molecule (e.g., a sulfonamido or carboxamido group) as found in the ammonium or organo-ammonium salts of this invention.

In U.K. Published Patent Application Nos. 2,051,831 A and 2,053,902 A the formation of the cyclized product

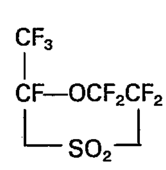

is reported. This cyclized product is an ether sulfone, not a cyclic anhydride. Said U.K. Published Patent Applications also disclose fluorinated cation exchange membranes which are prepared from certain

intermediates. The intermediates have the formula $FSO_2(CF_2)_nY$ where Y is —$COY^1$ or —CN [$Y^1$ is a halogen, hydrogen, —$NH_2$—, —OM (M is hydrogen, a metal or ammonium group), or —$OR^3$ ($R^3$ is an alkyl having 1 to 10 carbon atoms or an aryl)]; and n is an integer of 2 to 4.

Disclosure of invention

The present invention provides, in one aspect, cyclic anhydrides of perfluoroacids, characterized in that said perfluoroacids are selected from *omega*-sulfoperfluoroaliphaticcarboxylic acids and perfluoroaliphaticdisulfonic acids, and said cyclic anhydrides have the formula:

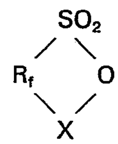

I

wherein $R_f$ is perfluoroalkylene having 2 to 5 backbone or catenary carbon atoms or perfluorocycloalkylene having 4 to 7, preferably 6, ring atoms, $R_f$ optionally being substituted by one or more, e.g., one to three, straight, branched, or cyclic perfluoroalkyl groups of 1 to 12, and preferably 1 to 4 carbon atoms, with $R_f$ having a total of up to 14 carbon atoms, and X is a carbonyl or sulfonyl radical. Preferably $R_f$ has the formula $-(CF_2)_m-$ where m is 2 to 4.

The present invention also provides a process for the preparation by ring formation of said cyclic anhydrides comprising the steps of:

(a) mixing *omega*-sulfoperfluoroaliphaticcarboxylic acid or perfluoroaliphaticdisulfonic acid precursor with excess phosphorus pentoxide,

(b) heating the resulting mixture to dehydrate and cyclize said acid precursor under anhydrous conditions, and

(c) recovering said cyclic anhydride under anhydrous conditions from the resulting reacted mixture.

The invention further provides carboxamide and sulfonamide derivatives of said cyclic anhydrides, which carboxamides and sulfonamides are useful as latent catalysts for the polymerization of cationically-sensitive monomers. Said carboxamides and sulfonamides are prepared by reacting one or more of said cyclic anhydrides with one or more protonic nitrogenous base having a $pk_b$ of about 13.2 or less. The preferred amide catalysts comprise carboxamides, sulfonamides, and mixtures thereof having the formulae:

$$R^1R^2N\overset{O}{\overset{\|}{C}}R_fSO_3^-\overset{+}{H_2}NR^1R^2 \qquad \text{II}$$

$$R^1R^2NSO_2R_fCO_2^-\overset{+}{H_2}NR^1R^2 \qquad \text{III}$$

$$R^1R^2NSO_2R_fSO_3^-\overset{+}{H_2}NR^1R^2 \qquad \text{IV}$$

wherein:

$R_f$ is as defined above;

each $R^1$ and $R^2$ is independently hydrogen, or a monovalent organic radical (preferably alkyl, alkyloxy, alkenyl, cycloalkyl, aryl or aryloxy, having 1 to 10 carbon atoms) which can be the same as or different from any other $R^1$ or $R^2$, or each $R^1$ and $R^2$ bonded to the same N atom can combine with one another to form a cyclic structure containing the N atom, and $R^1$ and $R^2$ can contain from 1 to 20 carbon atoms, can be straight chain, branched or cyclic, can be saturated, unsaturated or aromatic, can contain skeletal or catenary hetero atoms, i.e., atoms other than carbon (e.g., oxygen or sulfur), and can be unsubstituted or substituted with non-interferring moieties, i.e., moieties which do not interfere with the functioning of said amides as latent acid catalysts. Compounds of formulae II and IV above are especially preferred.

This invention also provides curable compositions comprising cationically-sensitive monomers and a catalytically effective amount of said cyclic anhydrides or said carboxamide or sulfonamide derivatives thereof.

This invention also provides a process for the polymerization of cationically-sensitive monomers, comprising the steps of:

(a) mixing with said monomers a catalytically effective amount of said cyclic anhydride or said carboxamide or sulfonamide derivative thereof, thereby forming a mixture, and

(b) allowing said mixture to polymerize, or heating said mixture to effect polymerization thereof.

Detailed description

In the practice of the present invention, said cyclic anhydrides are preferably prepared by the dehydration and cyclization of the precursor perfluoroacid hydrate, caused by heating the precursor acid in

the presence of an excess of a suitable dehydrating agent, e.g., phosphorus pentoxide, as shown below in Equation 1 (wherein the precursor perfluoroacid hydrate is an *omega*-sulfoperfluoroaliphaticcarboxylic acid, V, and the product, VI, is a compound of formula I wherein X is a carbonyl radical) and Equation 2 (wherein the precursor perfluoroacid hydrate is a perfluoroaliphaticdisulfonic acid, VII, and the product, VIII, is a compound of formula I wherein X is a sulfonyl radical). The dehydration and cyclization reaction is desirably carried out at a temperature sufficient to provide efficient and controllable reaction between the precursor acid and phosphorus pentoxide. For the reaction of Equation 1, such temperature is preferably about 100°C to 300°C and most preferably is about 150°C to 250°C. For the reaction of Equation 2, such temperature is preferably about 100° to 180°C.

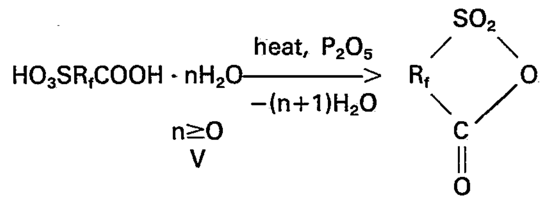


V

VI

Equation 1

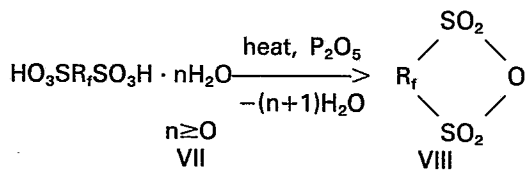


VII

VIII

Equation 2

The resulting cyclic anhydride product is volatile and can be collected by distillation. The cyclic anhydride is prepared and stored under anhydrous conditions.

The amount of phosphorus pentoxide can vary depending on the amount of water of hydration present in the precursor perfluoroacid hydrate. Generally, a one mole excess of phosphorus pentoxide is used with anhydrous precursor perfluoroacid, but greater amounts such as up to a ten mole excess or more can be used with hydrates of the precursor perfluoroacid.

An inert diluent such as sand, glass beads, or a high boiling fluorinated organic liquid is usually employed in the dehydration and cyclization reaction. The use of an inert, fluorinated organic liquid diluent having a boiling point which is substantially higher (e.g., 50°C) than the boiling point of the desired cyclic anhydride product is preferred since such a diluent facilitates intimate contact of the reactants, efficient stirring, and good heat transfer, thus aiding in the rapid completion of the dehydration and cyclization reaction.

When a fluorinated diluent is used, the volatile cyclic anhydride products can be isolated most conveniently on a small scale by purging the heated reaction flask with nitrogen gas and condensing the anhydride in a receiver cooled with "Dry Ice" to −78°C. Alternatively, the reaction mixture can be subjected to distillation.

The crude cyclic anhydride, obtained by any of the above procedures, can be additionally purified by redistillation, or can be used directly as an intermediate in the preparation of said carboxamide and sulfonamide derivatives. Said carboxamide and sulfonamide derivatives will sometimes be collectively referred to hereafter as "amide derivatives". The cyclic anhydride should be stored in a sealed dry vessel to avoid hydrolysis through contact with water or water vapor until it is needed for use as a catalyst or for the preparation of amide derivatives. The presence of water or water vapor can cause the cyclic anhydride to hydrolyze and form the precursor linear perfluoroacid hydrate.

The *omega*-sulfoperfluoroaliphaticcarboxylic acid precursors (Formula V, above) for the preparation of cyclic anhydrides of Formula VI, above, can be obtained by means of a series of reactions, starting with the conversion of hydrocarbon sultones or hydrocarbon *omega*-fluorosulfonylaliphaticacyl fluorides to the corresponding *omega*-fluorosulfonylperfluoroaliphaticacyl fluorides, $FSO_2R_fCOF$, by electrochemical fluorination in anhydrous hydrogen fluoride in accordance with the procedure described in U.S. Patent No. 2,732,398. Alkaline hydrolysis of said *omega*-fluorosulfonylperfluoroaliphaticacyl fluoride is usually performed using aqueous alkali metal hydroxide such as sodium or potassium hydroxide. A convenient method for preparation of the sodium salt involves the gradual addition of said *omega*-fluorosulfonylperfluoroaliphaticacyl fluoride to a stirred aqueous alcoholic solution (e.g., 80 to 85% by volume methanol) at room temperature, followed by neutralization with aqueous sodium hydroxide.

The resulting neutralized mixture is filtered to remove insoluble sodium fluoride and the filtrate evaporated yielding the salt, $NaSO_3R_fCOONa$, which is further dried by heating to 100°C.

The recovered metal salt, $M^1SO_3R_fCOOM^1$ (where $M^1$ is, for example, Na or K), is dissolved in water, and the resulting solution is placed on a column of cationic ion-exchange resin in the acid form (e.g., "Amberlite IR-120," commercially available from Rohm & Haas, Inc.). The column is eluted with distilled or deionized water, and the eluate is concentrated under reduced pressure at about 50°C to constant weight to yield the desired perfluoroacid hydrate precursor for the preparation of said cyclic anhydrides of Formula VI, above.

The perfluoroaliphaticdisulfonic acid precursors (Formula VII, above) for the preparation of cyclic anhydrides of Formula VIII, above, can be obtained by means of a series of reactions, starting with the conversion of aliphaticdisulfonyl fluorides, $R_h(SO_2F)_2$ (where $R_h$ is the hydrocarbon analog of said $R_f$ radical) to the corresponding perfluoroaliphaticdisulfonyl fluorides, $R_f(SO_2F)_2$, by electrochemical fluorination in anhydrous hydrogen fluoride in accordance with the procedure described in U.S. Patent No. 2,732,398. Alkaline hydrolysis of said perfluoroaliphaticdisulfonyl fluoride is performed by gradual addition thereof to a stirred solution of aqueous metal base such as carbonate or hydroxide of a metal such as sodium or potassium. Stirring is continued until completion of the reaction, followed by collection of the resulting solid salt product, rinsing of the salt with a small amount of cold water, and drying. The recovered product, $R_f(SO_3M^2)_2$ (where $M^2$ is for example, Na or K), is dissolved in water, and the resulting solution is placed on a column of cationic ion exchange resin in the acid form (e.g., "Amberlite IR-120"). The column is eluted with distilled or deionized water, and the eluate is concentrated under reduced pressure at about 50°C to constant weight to yield the desired acid hydrate precursor for the preparation of said cyclic anhydrides of formula VIII, above.

Representative cyclic *omega*-sulfoperfluoroaliphaticcarboxylic acid anhydrides of this invention include the following compounds:

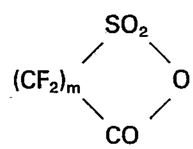

m=2, 3, or 4

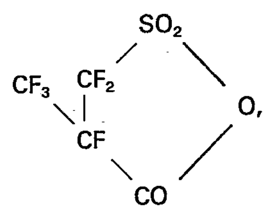

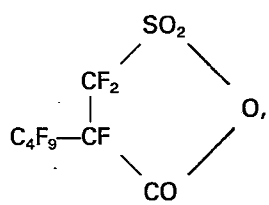

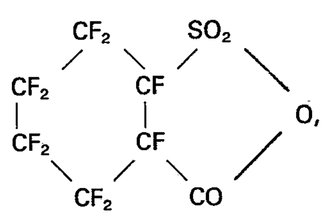

and

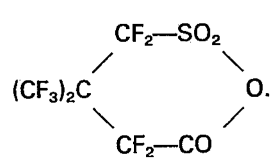

Representative cyclic perfluoroaliphaticdisulfonic acid anhydrides of this invention include the following compounds:

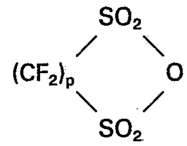

p=2, 3, 4 and 5

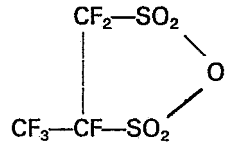

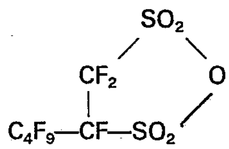

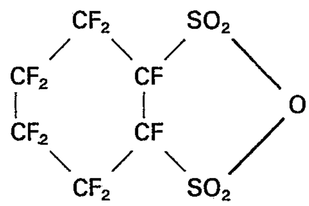

and

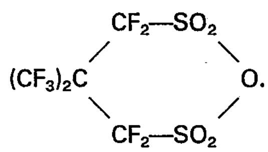

The amide derivatives of the cyclic anhydrides of the invention are obtained by reaction of the cyclic anhydride with a stoichiometric or excess amount of protonic nitrogenous base having a $pk_b$ of about 13.2 or less, such as ammonia, hydrazines, and organic amines containing at least one reactive hydrogen atom attached to nitrogen. The term "hydrazine" as used herein broadly includes hydrazine and hydrazine derivatives in which one or more hydrogen atoms bonded to nitrogen is replaced with $R^1$ or $R^2$ organic groups. Preferred nitrogenous bases have the formula $HNR^1R^2$, where $R^1$ and $R^2$ are as defined above. The cyclic anhydrides of this invention are reactive even with weakly basic amines such as diphenylamine (which has a $pk_b$ of 13.12).

The reaction of the cyclic anhydride with the protonic nitrogenous base is carried out at a temperature which provides efficient and controlled reaction between the cyclic anhydride and nitrogenous base. Such temperature is generally about −30° to 150°C, depending on the reactivity of the nitrogenous base. The reaction usually occurs readily at room temperature, with cooling sometimes being desirable to control the exotherm. The reactants can be combined in any order but a preferred method of conducting the amide formation reaction is by the slow addition of ammonia or amine to a stirred, cold (e.g., 0° to 10°C) solution of the cyclic anhydride in an anhydrous inert solvent such as methylene chloride. Other suitable inert solvents include diethyl ether, isopropyl ether and acetonitrile. The amide derivative is a salt in the form of an oil, grease, or solid. The oily or greasy salts are purified by decanting or evaporation of solvent. The solid salts can be isolated by filtration and purified by crystallization from an appropriate solvent or solvent mixture.

Preferred amide derivatives are obtained by the reaction of the cyclic anhydride with ammonia or a primary or secondary organic amine.

When the cyclic anhydride is derived from *omega*-sulfoperfluoroaliphaticcarboxylic acid, two products are formed. The major amide derivative formed is represented by Formula II above and can be referred to as the carboxamidoperfluoroaliphaticsulfonate. The minor amide derivative formed is represented by Formula III above and can be referred to as the sulfonamidoperfluoroaliphaticcarboxylate. Generally only small amounts of said sulfonamidoperfluoroaliphaticcarboxyate are obtained. However, when the protonic nitrogenous base used in the preparation of amide derivatives is ammonia, then a 1:1 mixture of compounds of Formulas II and III above is generally obtained.

When the cyclic anhydride is derived from perfluoroaliphaticdisulfonic acid, a single sulfonamide derivative is formed. This sulfonamide derivative is represented by Formula IV above and can be referred to as the sulfonamidoperfluoroaliphaticsulfonate.

Representative organic radicals $R^1$ and $R^2$ include methyl, ethyl, butyl, dodecyl, octadecyl, phenyl, *o*-tolyl, cyclopentyl, cyclohexyl, isopropyl, 2-ethylhexyl, propenyl, 2-butenyl, methoxymethyl, methoxyethyl, ethoxyethyl, ethoxybutyl, 4-methoxyphenyl, and eththioethyl. $R^1$ and $R^2$ together with N can be, for example, N-piperidyl or N-pyrrolidyl.

Representative organic amines having a $pk_b$ of about 13.2 or less and the formula $HNR^1R^2$ are described in "Handbook of Chemistry and Physics", 47th Edition, D-85 (1966—1967). Examples include methylamine, n-butylamine, n-octylamine, isobutylamine, cyclohexylamine, diethylamine, dioctylamine, diisobutylamine, diallylamine, glycine and its ethyl ester, aniline, N-methylaniline, *p*-chloroaniline, *p*-cyanoaniline, *o*-toluidine, *m*-aminophenol, diphenylamine, *alpha*-naphthylamine, morpholine, oxazolidine, thiazolidine, *p*-methoxyaniline, and the like. Such amines can contain substituent groups which are essentially non-reactive or less reactive than the amino group of the organic amine, including halogen, hydroxy, alkoxy, nitrile, carboxy and carboalkoxy.

Other protonic nitrogenous bases containing more than one basic —NH— or —$NH_2$— group can afford amide derivatives of this invention which are useful as catalysts. Such derivatives are generally more complex than the products represented by Formulae II, III, and IV, and can be oligomers or polymers. Such nitrogenous bases include hydrazine, sym-dimethylhydrazine, methylhydrazine, methylhydrazinecarboxylate, guanidine, aminoguanidine, diethylenetriamine, triethylenetetramine, hexamethylenediamine, piperazine, polyethyleneimine, and the like.

Representative simple (i.e., non-oligomeric) amide derivatives of the cyclic anhydrides of this invention include the following:

$$H_2NSO_2CF_2CF_2CF_2CO_2^-\overset{+}{N}H_4,$$

$$H_2NC(O)CF_2CF_2CF_2SO_3^-\overset{+}{N}H_4,$$

$$CH_3NHSO_2CF_2CF_2CF_2CF_2CO_2^-H_3\overset{+}{N}CH_3,$$

$$CH_3NHC(O)CF_2CF_2CF_2CF_2SO_3^-H_3\overset{+}{N}CH_3,$$

$$(C_2H_5)_2NSO_2CF_2CF_2CO_2^-H_2\overset{+}{N}(C_2H_5)_2,$$

$$(C_2H_5)_2NC(O)CF_2CF_2SO_3^-H_2\overset{+}{N}(C_2H_5)_2,$$

$$(HOC_2H_4)_2NSO_2CF_2CF(C_3F_7)CF_2CO_2^-H_2\overset{+}{N}(C_2H_4OH)_2,$$

$$(HOC_2H_4)_2NC(O)CF_2CF(C_3F_7)CF_2SO_3^-H_2\overset{+}{N}(C_2H_4OH)_2,$$

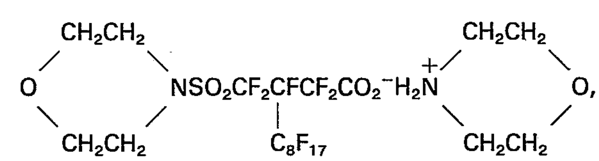
,

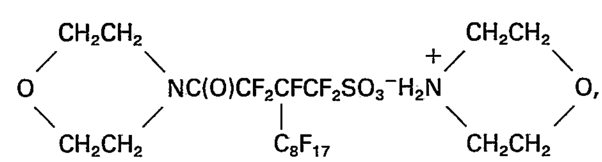
,

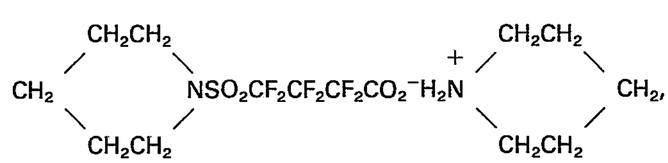
,

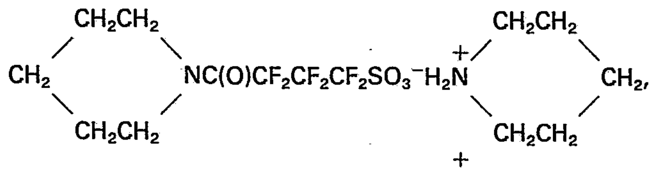

$C_6H_5N(CH_3)SO_2CF_2CF_2CF_2CO_2^{-}\,\overset{+}{H_2N}(CH_3)C_6H_5$,

$C_6H_5N(CH_3)C(O)CF_2CF_2CF_2SO_3^{-}\,\overset{+}{H_2N}(CH_3)_6H_5$,

$(i{-}C_3H_7)_2NSO_2CF_2CF_2CO_2^{-}\,\overset{+}{H_2N}(i{-}C_3H_7)_2$,

$(i{-}C_3H_7)_2NC(O)CF_2CF_2SO_3^{-}\,\overset{+}{H_2N}(i{-}C_3H_7)_2$,

$H_2NSO_2CF_2CF_2CF_2SO_3^{-}\,\overset{+}{N}H_4$,

$CH_3NHSO_2CF_2CF_2CF_2CF_2SO_3^{-}\,H_3\overset{+}{N}CH_3$,

$(C_2H_5)_2NSO_2CF_2CF_2SO_3^{-}\,\overset{+}{H_2N}(C_2H_5)_2$,

$(HOC_2H_4)_2NSO_2CF_2CF(C_3F_7)CF_2SO_3^{-}\,\overset{+}{H_2N}(C_2H_4OH)_2$,

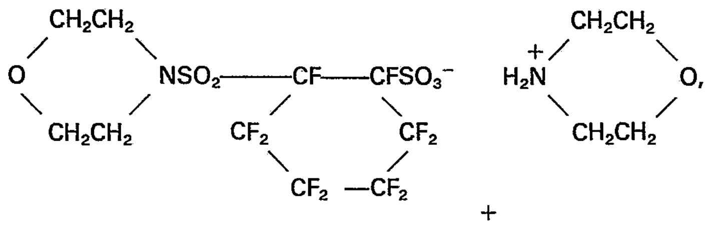

$C_6H_5N(CH_3)SO_2CF_2CF_2CF_2SO_3^{-}\,\overset{+}{H_2N}(CH_3)C_6H_5$,

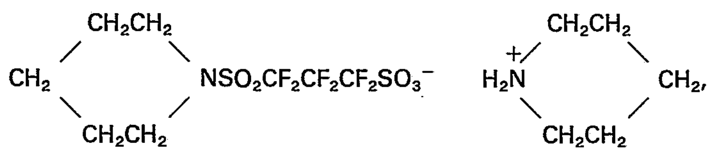

and

$(i{-}C_3H_7)_2NSO_2CF_2CF_2SO_3^{-}\,\overset{+}{H_2N}(i{-}C_3H_7)_2$.

The cyclic anhydrides of this invention and amide derivatives thereof (sometimes collectively referred to hereafter as the compounds of the invention) are useful for the polymerization or curing of cationically-sensitive monomers. The term "monomers" as used herein includes not only low molecular weight cationically-sensitive materials, but also high molecular weight polymeric compositions, e.g., resins containing one or more cationically-sensitive polymerizable groups of the types described below, which in the presence of the compounds of this invention will undergo polymerization or crosslinking.

Said amide derivatives of the cyclic anhydrides of this invention are latent catalysts, particularly with respect to epoxides. The term "latent catalyst" as used herein means a catalyst which does not exhibit or manifest any substantial curing or catalytic effect on monomer admixed therewith during normal storage or handling of such mixtures until the mixture is subjected to heat for the purpose of activation, though some small or otherwise tolerable or insignificant curing of the monomer may take place before activation, as evidenced by a slight increase in viscosity. Similarly, a composition which has latency or is characterized as being latently curable is one which during the period prior to being heated to effect cure, exhibits little if any gelling, polymerization, etc., though some small or otherwise tolerable or insignificant curing may take place during such period.

The monomers that can be cured or polymerized with the compounds of this invention, using the latter in a catalytically effective amount, are those known to undergo cationic polymerization and include 1,2-, 1,3-, and 1,4-cyclic ethers (also designated as 1,2-, 1,3-, and 1,4-epoxides), vinyl ethers, N-vinyl compounds, ethylenically unsaturated hydrocarbons, cyclic formals, and cyclic organosiloxanes. An extensive list of cationically polymerizable monomers which can be used in this invention is given in U.S. Patent Nos. 3,347,676 and 3,842,019.

The cyclic ethers which can be polymerized in accordance with this invention include those described

in "Ring-Opening Polymerizations," Vol. 2, by Frisch and Reegan, Marcel Dekker, Inc. (1969). Suitable 1,2-cyclic ethers are the monomeric and polymeric types of epoxides. They can be aliphatic, cycloaliphatic, aromatic, or heterocyclic and will typically have an epoxy equivalency of from 1 to 6, preferably 1 to 3. Particularly useful are the aliphatic, cycloaliphatic, and glycidyl ether type 1,2-epoxides such as propylene oxide, epichlorohydrin, styrene oxide, vinylcyclohexene oxide, vinylcyclohexene dioxide, glycidol, butadiene oxide, glycidyl methacrylate, diglycidyl ether of bisphenol A, 3,4-epoxycyclohexylmethyl 3,4-epoxycyclohexanecarboxylate, 3,4-epoxy-6-methylcyclohexylmethyl 3,4-epoxy-6-methylcyclohexanecarboxylate, bis(3,4-epoxy-6-methylcyclohexylmethyl) adipate, dipentene oxide, epoxidized polybutadiene 1,4-butanediol diglycidyl ether, polyglycidyl ether of phenolformaldehyde resole or novolak resin, resorcinol diglycidyl ether, and epoxy silicones, e.g., dimethylsiloxanes having cycloaliphatic epoxide or glycidyl ether groups. A wide variety of commercial epoxy resins is available and listed in "Handbook of Epoxy Resins" by Lee and Neville, McGraw-Hill Book Company, New York (1967) and in "Epoxy Resin Technology" by P. F. Bruins, John Wiley & Sons, New York (1968). Representative of the 1,3- and 1,4- cyclic ethers which can be polymerized in accordance with this invention are oxetane, 3,3-bis(chloromethyl)oxetane, and tetrahydrofuran.

Another useful class of cationically-sensitive monomers which can be polymerized in accordance with this invention is represented by the general formula $CH_2=C(Y)XR'$, where X is —O— or —NR''— (where R'' is hydrogen or lower alkyl), R' is hydrocarbyl, hydrocarbylcarbonyl, halohydrocarbyl, or hydroxyhydrocarbyl when X is oxygen, or R' is hydrocarbyl, hydrocarbylcarbonyl, or hydrocarbylsulfonyl when X is nitrogen, and Y is hydrogen, alkyl, aryl, or other hydrocarbyl, or R' (as hydrocarbylcarbonyl) and R'' can be connected to form a 5- or 6-membered cyclic structure containing nitrogen as a hetero ring atom. The term "hydrocarbyl" is used herein in its usual sense to mean alkyl, alkenyl, aryl, cycloalkyl, cycloalkenyl, alkaryl, arylalkyl, and the like. In general, monomers of this type contain a vinyl group and are typified by vinyl alkyl ethers, such as vinyl methyl ether, vinyl ethyl ether, vinyl n-butyl ether, vinyl 2-chloroethyl ether, vinyl isobutyl ether, vinyl phenyl ether and vinyl 2-ethylhexyl ether, vinyl ethers of substituted aliphatic alcohols such as divinyl ether of butanediol, hydroxybutyl vinyl ether, and N-vinyl compounds such as N-vinyl-N-methyl octanesulfonamide and N-vinylpyrrolidone. A description of vinyl monomers and their use in preparing polymers is set forth in "Vinyl and Related Polymers," by Schildknecht, published by John Wiley & Sons, Inc., New York (1952).

Other cationically-sensitive monomers which can be polymerized in this invention include ethylenically unsaturated hydrocarbons such as isobutylene, 1,3-butadiene, isoprene, styrene, and divinylbenzene, especially the vinyl benzenes, cyclic formals such as trioxane, 1,3-dioxolane, 2-vinyl-1,3-dioxolane and methyl-1,3-dioxolane, and cyclic siloxanes which can contain various groups attached to the silicon atom such as a hydrocarbon radical (alkyl, aryl, alkaryl), an alkenyl hydrocarbon radical (vinyl, allyl or acryloyloxyalkyl), a halogenated hydrocarbon radical, a carboxy-containing hydrocarbon radical or ester group, a cyanohydrocarbon radical, hydrogen, halogen or a hydroxy group. Representative cyclic siloxanes are hexamethylcyclotrisiloxane, octamethylcyclotetrasiloxane, phenylheptamethylcyclotetrasiloxane, vinylheptamethylcyclotetrasiloxane, methacryloyloxymethylheptamethylcyclotetrasiloxane, bromomethyl-heptamethylcyclotetrasiloxane, 3-chloropropylheptamethylcyclotetrasiloxane, 1,2,3-tris(3,3,3-trifluoropropyl)-1,2,3-trimethylcyclotrisiloxane, acetoxymethylheptamethylcyclotetrasiloxane, cyanomethyl-heptamethylcyclotetrasiloxane, 1,2,3-trihydro-,1,2,3-trimethylcyclotrisiloxane, and chloroheptamethyl-cyclotetrasiloxane. Other known cyclic siloxanes are listed in "Chemistry and Technology of Silicones" by Walter Noll, Academic Press, New York (1968), Tables 41, 44 and 45.

The cyclic siloxanes can also be polymerized in the presence of relatively low molecular weight linear siloxanes such as hexamethyldisiloxane, chloropentamethyldisiloxane and octamethyltrisiloxane which serve to terminate the growing chain and provide stable fluids or fluids having reactive end groups.

There is a host of commercially available cationically-sensitive monomers which can be used in this invention. In particular, cyclic ethers which are readily available include propylene oxide, oxetane, epichlorohydrin, tetrahydrofuran, styrene oxide, vinylcyclohexene oxide, glycidol, glycidyl methacrylate, octylene oxide, phenyl glycidyl ether, 1,2-butane oxide, diglycidyl ether of bisphenol A (e.g., "Epon 828" and "DER 331"), vinylcyclohexene dioxide (e.g., "ERL-4206"), 3,4-epoxycyclohexylmethyl-3,4-epoxycyclohexanecarboxylate (e.g., "ERL-4221"), 3,4-epoxy-6-methylcyclohexylmethyl-3,4-epoxy-6-methylcyclohexanecarboxylate (e.g., "ERL-4201"), bis(3,4-epoxy-6-methylcyclohexylmethyl)-adipate (e.g., "ERL-4289"), aliphatic epoxy modified with polypropylene glycol (e.g., "ERL-4050" and "ERL-4052"), dipentene dioxide (e.g., "ERL-4269"), epoxidized polybutadiene (e.g., "Oxiron 2001"), silicone epoxy (e.g., "Syl-Kem 90"), 1,4-butanediol diglycidyl ether (e.g., "Araldite RD-2"), polyglycidyl ether of phenolformaldehyde novolak (e.g., "DEN-431", "Epi-Rez 521" and "DEN-438"), resorcinol diglycidyl ether (e.g., "Kopoxite"), polyglycol diepoxide (e.g., "DER 736"), polyacrylate epoxide (e.g., "Epocryl U-14"), urethane modified epoxide (e.g., "QX3599"), polyfunctional flexible epoxides (e.g., "Flexibilizer 151"), and mixtures thereof as well as mixtures thereof with co-curatives, curing agents, or hardeners which also are well known (see Lee and Neville and Bruins, supra). Representative of the co-curatives or hardeners which can be used are acid anhydrides such as nadic methyl anhydride, cyclopentanetetracarboxylic dianhydride, pyromellitic anhydride, cis-1,2-cyclohexanedicarboxylic anhydride, and mixtures thereof.

In general, the polymerization of cationically-sensitive monomers with the cyclic anhydrides of this invention can be carried out at room temperature for the majority of cationically-sensitive monomers, although low temperature (e.g., −10°C) or elevated temperatures (e.g., 30° to 200°C, preferably 50° to 100°C), can be used to either subdue the exotherm of polymerization or to accelerate the polymerization. In the case of latent amide catalysts of this invention, temperatures generally in the range of 50° to 250°C, preferably from 80° to 150°C, can be used. The temperature of polymerization and amount of catalyst will vary and be dependent on the particular cationically-sensitive monomer used and the desired application of the polymerized or cured product.

The amount of cyclic anhydride or amide derivative thereof to be used as a catalyst in this invention (i.e., a catalytically effective amount) should be sufficient to effect polymerization of the cationically-sensitive monomer under the desired use conditions. Such amount generally will be in the range of about 0.01 to 20 weight percent, preferably 0.5 to 5 weight percent, and most preferably 1 to 2 weight percent, based on the weight of cationically-sensitive monomer.

Solvents can be used to assist in dissolution of the cyclic anhydride or amide derivative thereof in the cationically-sensitive monomer, and are preferred for use with amide derivatives. Representative solvents include acetone, methylene chloride, ethyl acetate, methyl ethyl ketone, acetonitrile, *p*-dioxane, and the dimethyl ether of ethylene glycol (glyme). In general, in compositions containing cyclic anhydride catalysts, basic solvents or basic impurities in the monomer are avoided to prevent deactivation of the anhydride catalyst.

The curable or polymerizable compositions of this invention, consisting of or consisting essentially of the cationically-sensitive monomer(s) and said cyclic anhydride or amide derivative thereof as catalyst, can be used for applications like those cationically-sensitive monomer systems cured with other catalysts, such as epoxides cured with $BF_3$ or the complex of $BF_3$ with diethyl ether. Also, curable compositions of the invention comprising cationically-sensitive monomer(s), said cyclic anhydride or amide derivative thereof as catalyst, and other adjuvants (e.g., fillers, reinforcements, pigments, extenders, plasticizers and surface modifying agents) can be prepared in the same manner as compositions containing cationically-sensitive monomers, other catalysts, and adjuvants. For example, the curable compositions of this invention can be used as adhesives, caulking and sealing compounds, casting and molding compounds, potting and encapsulating compounds, impregnating and coating compounds, etc., depending on the particular monomers and/or catalyst used. Where the catalyst is used in its latent form, the curable composition can be used as a one-component or cured-in-place system, such capability enhancing its use for the applications mentioned above. One particular application where such capability can be employed is in the electrical arts, where such latently curable compositions can be used to coat or impregnate for insulation or protective purposes electrical motor windings or coils, transformers, capacitors, electrical terminals, cables, and other electrical devices.

The curable epoxy composition of this invention can be used to make shaped articles of self-supporting, structural, filled or reinforced epoxy resin composites, such as glass fiber cloth reinforced epoxy resin composites, useful, for example, as repair materials. The various fillers, reinforcements, and other particulate materials to be mixed or coated with or dispersed in the curable compositions of this invention to make the composites of this invention, as well as methods of processing these materials in making the composites, and their applications, are those known to the art. In this connection, reference is made to "Modern Composite Materials," edited by Brautman and Krock, published by Addison-Wesley Publishing Company, Reading, Mass. (1967); and "Handbook of Fiberglass and Advanced Plastics Composites," edited by G. Lubin, published by Van Nostrand Reinhold Company, New York, N.Y. (1969).

The objects and advantages of this invention are further illustrated by the following examples, but the particular materials and amounts thereof recited in these examples as well as other conditions and details should not be construed to unduly limit this invention.

Examples 1 to 8 illustrate the preparation of cyclic anhydrides of this invention.

Example 1

To a stirred solution of 300 ml of methanol and 50 ml of water, cooled with an ice-water bath, was slowly added 50.2 g of 4-fluorosulfonylperfluorobutyryl fluoride. An aqueous base solution containing about 23 g of sodium hydroxide and 23 ml of water was then added with cooling. Additional aqueous base solution was added until the reaction mixture was neutral to pH paper. The reaction mixture was stirred for two hours and filtered to remove sodium fluoride. The filtrate was distilled to remove volatiles and the residue dried on a steam bath giving 51.3 g of the disodium salt of 4-sulfoperfluorobutyric acid, in the form of a white solid. About 20 g of the sodium salt was dissolved in water and ion-exchanged using "Amberlite IR-120" ion-exchange resin. The acidic aqueous solution was evaporated on a steam bath, yielding 16 g of the hydrate of 4-sulfoperfluorobutyric acid.

4-Sulfoperfluorobutyric acid (7.8 g, azoeotropically dried with toluene) was mixed in a flask under nitrogen with 15 g of phosphorus pentoxide. The flask was equipped for distillation under anhydrous conditions and heated with a sand bath to a temperature of 260°C, giving a distillate of 3.2 g of the cyclic anhydride of 4-sulfoperfluorobutyric acid, b.p. 76—78°C. The identity of the anhydride was established by infrared, fluorine nuclear magnetic resonance (Fnmr), and mass spectral analyses. The anhydride is highly reactive, e.g., reacts with moist air, and is stored under anhydrous conditions.

Example 2

In a 500 ml three-necked flask fitted with a mechanical stirrer, thermometer, condenser and addition funnel, and containing a cooled solution of 84.9 g (1.52 mol) of KOH dissolved in 84.9 g water, was added gradually over about 0.5 hour, at a rate so that the reaction temperature did not exceed about 35°C, 69.3 g (0.30 mol) of $FO_2SCF_2CF_2COF$ (from the electrochemical fluorination of

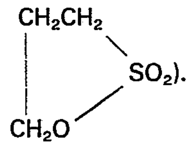

Stirring under ambient conditions was continued for about 0.5 hour. The temperature was next raised to about 100°C and stirring continued under these conditions for four hours. The reaction mixture was cooled and the solid crystalline product filtered, rinsed with 50 ml cold water, and the crystals dried under reduced pressure. The yeild of $KO_3SCF_2CF_2COOK$ salt was 52 g.

Ten grams of $KO_3SCF_2CF_2COOK$ were dissolved in 30 ml warm water and placed in a 50 cm×2.5 cm glass column containing a 20 cm bed of "Amberlite IR-120" ion-exchange resin in the acid ($H^+$) form, which had been previously prepared by treating the column with 6N hydrochloric acid and rinsing with distilled water. The column was eluted with distilled water. The first 150 ml of eluate was concentrated under reduced pressure to yield the hydrate of 3-sulfoperfluoropropionic acid, $HO_3SCF_2CF_2COOH \cdot 8H_2O$.

To a stirred mixture of 30 g of phosphorus pentoxide in 50 ml of tris(perfluoroamyl)amine in a 250 ml, three-necked flask fitted with a thermometer, mechanical stirrer and anhydrous nitrogen gas inlet, and connected to a trap cooled to −78°C with "Dry Ice", was added 5.0 g of $HO_3SCF_2CF_2COOH \cdot 8H_2O$.

The reaction mixture was heated over a 1.5 hour period to a maximum of 165°C while stirring and purging with a slow stream of nitrogen gas. Warming the −78°C trap gave 3.2 g of a colorless liquid, b.p. 54—56°C. The infrared spectrum of the liquid was consistent with the structure:

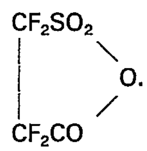

Example 3

Using the method of Example 1, 50 g of a mixture containing about 75 mol percent 4-fluorosulfonylperfluorobutyryl fluoride and about 25 mol percent of a mixture of 3-fluorosulfonyl-2-trifluoromethylperfluoropropionyl fluoride and 3-fluorosulfonyl-3-trifluoromethylperfluoro-propionyl fluoride was hydrolyzed with aqueous sodium hydroxide, giving 52.3 g of dry sodium salt mixture. The Fnmr spectrum indicated the presence of a mixture of about 75 mol% of the disodium salt of 4-sulfoperfluorobutyric acid and about 12 mol % each of the disodium salts of the isomeric acids 3-sulfo-2-trifluoromethylperfluoropropionic acid and 3-sulfo-3-trifluoromethylperfluoropropionic acid. The sodium salt mixture (20 g) was ion-exchanged giving 16 g of a mixture of the hydrates of the above sulfoperfluoroalkanecarboxylic acids.

Mixing of 8.3 g of the above acid hydrates and 17 g of phosphorus pentoxide in a flask followed by heating in a sand bath to a temperature of 250°C gave 3.5 g of distillate (b.p., 70—75°C) which contained the cyclic anhydride of 4-sulfoperfluorobutyric acid as the major component along with about 10 mol % each of the cyclic anhydrides of 3-sulfo-2-trifluoromethylperfluoropropionic acid and 3-sulfo-3-trifluoromethyl-perfluoropropionic acid. The structures of these latter two anhydride compounds are:

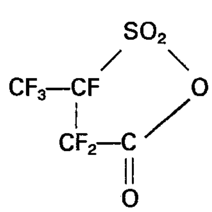 and 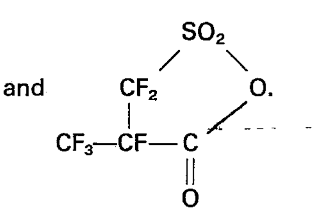

Example 4

To a 500 ml, three-necked flask fitted with a mechanical stirrer, thermometer, condenser and addition funnel, and containing 54.7 g (0.97 mole) of KOH dissolved in 100 ml water, was added gradually over about 1 hour, with heating (about 80°C) and stirring, 63.2 g (0.20 mole) of hexafluoro-1,3-propanedisulfonyl fluoride, $FO_2SCF_2CF_2CF_2SO_2F$. Heating and stirring were continued for about three more hours and the reaction mixture was allowed to cool overnight. The reaction product was filtered, washed with 25 ml of

cold water, and allowed to air dry, yielding 78 g of fine, white crystals. Infrared analysis of the crystals was consistent with the structure hexafluoro-1,3-propanedisulfonic acid dipotassium salt, $KO_3SCF_2CF_2CF_2SO_3K$.

A 9.7 g sample of the salt was dissolved in 50 ml of warm water and placed in a 50 cm×2.5 cm glass column containing a 20 cm bed of ion exchange resin ("Amberlite IR-120") in the acid ($H^+$) form which had been previously prepared by treating the resin with 6N hydrochloric acid and rinsing with distilled water. The column was eluted with distilled water. The first 100 ml of eluate were concentrated under reduced pressure to yield 7.5 g of clear liquid product. The identity of the product was established by infrared spectroscopy, fluorine NMR (Fnmr) analysis, and water determination, as the hexahydrate of hexafluoro-1,3-propanedisulfonic acid, $HO_3SCF_2CF_2CF_2SO_3H \cdot 6H_2O$. The acid hydrate partially crystallized on standing.

A mixture of 11.3 g (0.027 mole) of the above disulfonic acid hydrate (from another run) and 30 g of phosphorus pentoxide was heated to 130°C under reduced pressure in a flask adapted for short-path distillation. A total of 4.3 g colorless liquid product was collected in a trap cooled with "Dry Ice" to −78°C. The liquid product was redistilled, yielding a colorless, non-fuming liquid with a boiling range of 109—110°C at atmospheric pressure, $N^{22}_D$ 1.3562. The product is insoluble in water at room temperature, but hydrolyzes within a few minutes to give a homogeneous solution. Analytical data are consistent with the structure hexafluoro-1,3-propanedisulfonic acid anhydride,

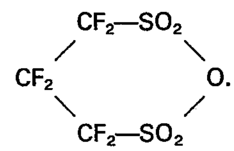

Example 5

This example illustrates another method for carrying out the dehydration and cyclization reaction of Equation 2, above, utilizing an inert, high-boiling, fluorinated organic diluent.

To a stirred mixture of 30 g of phosphorus pentoxide in 75 ml of tris(perfluoroamyl)amine, $(C_5F_{11})_3N$, in a 250 ml, three-necked flask fitted with a thermometer, mechanical stirrer and nitrogen gas inlet, and connected to a trap cooled with "Dry Ice" to −78°C, was added 5.0 g of hexafluoro-1,3-propanedisulfonic acid hexahydrate. The resulting mixture was heated over a one hour period to a maximum of 170°C while stirring and purging with a slow stream of nitrogen. Warming the −78°C trap gave a colorless liquid weighing 2.4 g (69% yield). The infrared spectrum was consistent with the desired cyclic hexafluoro-1,3-propanedisulfonic acid anhydride.

Example 6

Using the method of Example 4, cyclic tetrafluoro-1,2-ethanedisulfonic acid anhydride,

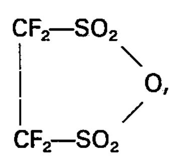

b.p. 100°C to 101°C, was prepared. The identity of the liquid anhydride product was established by infrared and Fnmr analysis. The anhydride fumes in moist air, hydrolyzing to form the disulfonic acid hydrate precursor.

Example 7

Using the method of Example 4, cyclic octafluoro-1,4-butanedisulfonic acid anhydride,

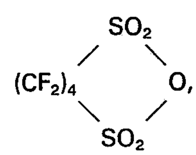

b.p. 126.5°—127.0°C, was prepared. The dehydration and cyclization reaction was carried out using sand in the reaction vessel.

Example 8

Using the method of Example 4, cyclic decafluoro-1,5-pentanedisulfonic acid anhydride,

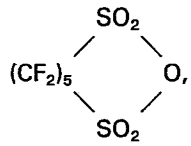

was prepared. The dehydration and cyclization reaction was carried out using sand in the reaction vessel.

The next six examples show the preparation of amide derivatives of cyclic anhydrides of this invention.

Example 9

Under anhydrous conditions, 1.9 g of the mixture of cyclic anhydrides of Example 3 was slowly added to a stirred solution of 1.9 g of diethylamine and 20 ml of diethyl ether in a 100 ml flask cooled with an ice-water bath. Formation of a lower phase, insoluble product occurred immediately. The mixture was stirred for 0.5 hour and the ether distilled off. The residue was heated with warm water under reduced pressure giving 2.8 g of an amber oily residue. Analyses of the oil by infrared and Fnmr indicated the presence of a mixture of

$$(C_2H_5)_2NC(O)(CF_2)_3SO_3^{-}H_2\overset{+}{N}(C_2H_5)_2,$$

$$(C_2H_5)_2NC(O)CF(CF_3)CF_2SO_3^{-}H_2\overset{+}{N}(C_2H_5)_2, \text{ and}$$

$$(C_2H_5)_2NC(O)CF_2CF(CF_3)SO_3^{-}H_2\overset{+}{N}(C_2H_5)_2.$$

Example 10

Using the method of Example 9, aniline (1.7 g) in 25 ml of diethyl ether was allowed to react with the anhydride of 4-sulfoperfluorobutyric acid (1.1 g), giving a white solid product. After stirring the mixture for 1 hr, 1.0 g of solid product was isolated by filtration. Analyses indicated high purity

$$C_6H_5NHC(O)(CF_2)_3SO_3^{-}H_3\overset{+}{N}C_6H_5.$$

Example 11

Under anhydrous conditions, 1.0 g of ammonia was slowly bubbled into a solution of the cyclic anhydride of 3-sulfoperfluoropropionic acid (Example 2, 1.0 g) which had been dissolved in acetonitrile. The resulting mixture was distilled under reduced pressure, giving 1.0 g of the ammonia reaction product which was a heavy grease soluble in acetone. Analyses by infrared and Fnmr indicated the presence of essentially a 1:1 mixture of

$$NH_2C(O)(CF_2)_3SO_3^{-}\overset{+}{N}H_4 \text{ and } NH_2SO_2(CF_2)_3CO_2^{-}\overset{+}{N}H_4.$$

Example 12

About 1.0 g of the cyclic hexafluoro-1,3-propanedisulfonic acid anhydride of Example 4 was added dropwise to a stirred 1.0 g portion of piperidine which had been cooled to −10°C. The dark crude solid product was recrystallized from an ethyl acetate/diethyl ether mixture to yield gold colored crystals, m.p. 86—88°C.

The structure of the mixed sulfonamide-sulfonate salt,

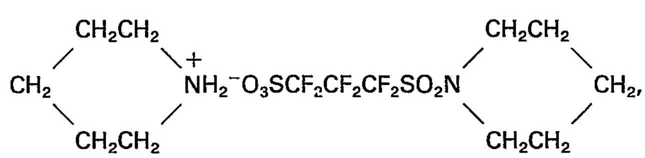

was established by infrared analysis.

Example 13

A reaction product with ammonia was prepared by the slow addition of excess ammonia gas to an anhydrous methylene chloride solution of the cyclic anhydride prepared in Example 4. The resulting solid product was crystallized from a mixture of diethyl ether and carbon tetrachloride to yield white crystals, m.p. 168.5—169°C. Elemental analysis, infrared analysis, and Fnmr spectra were consistent with the structure:

$$\overset{+}{N}H_4{}^{-}O_3SCF_2CF_2CF_2SO_2NH_2.$$

Example 14

A reaction product of the cyclic anhydride of Example 7 with ammonia was prepared by the gradual addition of ammonia gas to a methylene chloride solution of the cyclic anhydride. The product was a white powder. The mixed sulfonamide-ammonium salt structure,

$$NH_4^{+-}O_3SCF_2—CF_2—CF_2—CF_2—SO_2NH_2,$$

was established by infrared analysis and Fnmr spectra.

The next ten examples show the use of cyclic anhydrides of this invention as catalysts for the polymerization of cationically-sensitive monomers.

Example 15

Polymerization of tetrahydrofuran

The cyclic anhydride of 3-sulfoperfluoropropionic acid (0.04 g) was added under nitrogen to a glass vial containing 2.4 g of commercial tetrahydrofuran. The vial was capped and polymerization observed at room temperature. After about 20 hours a heavy grease was obtained. A portion of the polymer was stirred with water for four hours. Next, the insoluble polymer was removed and allowed to air dry. A high molecular weight, tough, leathery polymer of tetrahydrofuran was obtained.

Example 16

Polymerization of styrene

Using the method of Example 15, 0.04 g of the cyclic anhydride of 3-sulfoperfluoropropionic acid was added to 2.0 g of styrene. An immediate, very exothermic polymerization resulted. After 2 hours at 25°C, the resulting polymer was a brown, essentially non-flowable, soft solid.

Example 17

Polymerization of N-vinyl pyrrolidone

Using the method of Example 15, 0.05 g of the cyclic anhydride of 3-sulfoperfluoropropionic acid was added to 2.2 g of N-vinyl-2-pyrrolidinone. An immediate exothermic polymerization occurred and after five minutes at 25°C, the resulting polymer was an amber, heavy grease.

Example 18

Polymerization of an aliphatic diepoxide

Using the method of Example 15, 0.04 g of the cyclic anhydride of 3-sulfoperfluoropropionic acid was added to 3.9 g of "ERL 4221" epoxy resin. Immediate gelation occurred and after four hours at 25°C, a solid polymer dispersed in the epoxy liquid was obtained.

Example 19

Polymerization of octamethylcyclotetrasiloxane

Using the method of Example 15, 0.06 g of the cyclic anhydride of 3-sulfoperfluoropropionic acid was added to 3.3 g of octamethylcyclotetrasiloxane. After about three days at 25°C, a colorless, non-tacky gum characteristic of a very high molecular weight polydimethylsiloxane polymer was obtained.

Example 20

Polymerization of tetrahydrofuran

In a 500 ml resin flask fitted with mechanical stirrer, thermometer and nitrogen gas inlet was placed 100 g of anhydrous tetrahydrofuran and 15 g of cyclohexane. The flask contents were cooled to 4°C with an ice bath, then 2.0 g of hexafluoro-1,3-propanedisulfonic acid anhydride was added. A mildly exothermic polymerization reaction ensued, accompanied by increased viscosity of the reaction mixture. After one hour, the ice bath was removed. After three hours, stirring was very difficult. Additional cyclohexane (65 g) and 120 g of toluene were added to the reaction vessel and the resulting solution was mixed with 100 g of toluene which had been saturated with anhydrous ammonia. The resulting solution was stirred for one hour at toom temperature with 16.7 g of anion exchange resin ("Amberlite IRA-402"), then filtered to remove the resin particles. A sample of the —$NH_2$ terminated tetramethylene oxide polymer product, $H_2N(C_4H_8O)_nC_4H_8NH_2$, was isolated from solution and characterized by gel permeation chromatography.

Example 21

Polymerization of an aliphatic diepoxide

To a small glass vial containing a solution of 2.0 g of 3,4-epoxycyclohexylmethyl-3,4-epoxycyclohexanecarboxylate ("ERL 4221" epoxide) in 2 ml of methylene chloride was added one drop of the cyclic anhydride of Example 20. A small quantity of solid formed initially, but there was no apparent viscosity increase after two days at room temperature. Heating at about 130°C for 10 min. resulted in the formation of a dark, solid resinous product, useful as a potting resin.

Example 22

Polymerization of styrene

To a small glass vial containing a solution of 2.0 g of styrene in 2 ml of methylene chloride was added one drop of the cyclic anhydride of Example 20. After 0.75 hour, the solution was cloudy. After standing overnight, the solution was very viscous. A clear, tough polymer resulted after several days.

Example 23

Polymerization of N-vinyl pyrrolidone

Using the method of Example 22, one drop of the cyclic anhydride of Example 20 was added to a solution of 2.0 g of N-vinyl pyrrolidone in 2 ml of methylene chloride. A viscous oil was obtained after four days.

Example 24

Polymerization of Octamethylcyclotetrasiloxane

Using the method of Example 22, one drop of the cyclic anhydride of Example 20 was added to a solution of 2.0 g of octamethylcyclotetrasiloxane, $[(CH_3)_2SiO]_4$, in 2 ml of methylene chloride. The viscosity of the solution increased overnight. A solid polymer was obtained after four days.

The next four examples show the use of amide derivatives of cyclic anhydrides of this invention as latent catalysts for the polymerization of cationically-sensitive monomers.

Example 25

To a glass vial was added 0.03 g of the ammonia reaction product of Example 11 and 2.0 g of "ERL 4221" epoxy resin. The mixture was heated at 50°C for one minute giving a homogeneous yellow fluid. No evidence of polymerization was observed on storage of the fluid at room temperature for three days, indicating latency of the catalyst. The fluid was then heated at 125°C for 15 minutes giving a brown, non-flowable solid polymer, having utility as a potting compound.

Example 26

To a glass vial containing a solution of 0.04 g of the aniline reaction product of Example 10 and about 0.06 g of acetone was added 1.2 g of "ERL 4221" epoxy resin. The vial was capped and the mixture heated at 50°C for 1 minute giving a clear light yellow fluid. No evidence of polymerization was observed on storage of the fluid at room temperature for 20 hr, indicating latency of the catalyst. The fluid was then heated at about 125°C for 5 min. giving a hard, solid polymer.

Example 27

In a glass vial was placed about 25 mg of

$$H_2NSO_2CF_2CF_2CF_2SO_3^{-}\overset{+}{N}H_4$$

(from Example 13) and 1.0 g "ERL 4221" epoxide. The salt dissolved in the epoxide at room temperature. There was no apparent change in viscosity of the epoxide/salt solution after 30 hours at room temperature, but on heating at 130°C for 10 minutes, a clear, nearly colorless brittle solid formed.

Example 28

In a glass vial was placed about 25 mg of

$$CH_3NHSO_2CF_2CF_2CF_2SO_3^{-}\overset{+}{H_2}NCH_3$$

(prepared using the method of Example 13, but with $CH_3NH_2$ in place of $NH_3$) and 1.0 g of "ERL 4221" epoxide. The salt dissolved at room temperature yielding a pale amber solution. There was no apparent reaction or viscosity change after 30 hours at room temperature, but on heating at 130°C for 10 minutes, an amber, brittle solid was produced.

**Claims**

1. Cyclic anhydrides of perfluoroacids, characterized in that said perfluoroacids are selected from *omega*-sulfoperfluoroaliphaticcarboxylic acids and perfluoroaliphaticdisulfonic acids, and said cyclic anhydrides have the formula:

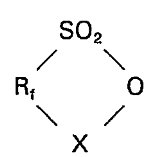

wherein $R_f$ is perfluoroalkylene having 2 to 5 catenary carbon atoms or perfluorocycloalkylene having 4 to 7 ring atoms, $R_f$ optionally being substituted by one or more straight chain, branched, or cyclic perfluoroalkyl groups of 1 to 12 carbon atoms, with $R_f$ having a total of up to 14 carbon atoms, and X is a carbonyl or sulfonyl radical.

2. Compounds according to Claim 1, further characterized in that $R_f$ is perfluoroalkylene having 2 to 5 catenary carbon atoms, $R_f$ optionally being substituted by one or more straight chain or branched perfluororalkyl groups of 1 to 4 carbon atoms.

3. Compounds according to Claim 1, further characterized in that $R_f$ is $-(CF_2)_m-$ and m is 2 to 4.

4. A compound according to Claim 3, further characterized in that m is 2.

5. A compound according to Claim 3, further characterized in that m is 3.

6. A compound according to Claim 3, further characterized in that m is 4.

7. Compounds according to any preceding claim, further characterized in that X is a carbonyl radical.

8. Compounds according to any of Claims 1 to 6, further characterized in that X is a sulfonyl radical.

9. Amides, characterized in that said amides are derivatives of said compounds according to Claim 1 and have the formulae:

$$R^1R^2N\overset{\overset{O}{\|}}{C}R_fSO_3^-H_2\overset{+}{N}R^1R^2,$$

$$R^1R^2NSO_2R_fCO_2^-H_2\overset{+}{N}R^1R^2, \text{ or}$$

$$R^1R^2NSO_2R_fSO_3^-H_2\overset{+}{N}R^1R^2$$

wherein the compounds are derived from amines of formula $HR^1R^2$ having a $pk_b$ of 13.2 or less, $R_f$ is perfluoroalkylene having 2 to 5 catenary carbon atoms or perfluorocycloalkylene having 4 to 7 ring atoms, $R_f$ optionally being substituted by one or more straight chain, branched, or cyclic perfluoroalkyl groups of 1 to 12 carbon atoms, with $R_f$ having a total of up to 14 carbon atoms, and each $R^1$ and $R^2$ is independently hydrogen, or a monovalent organic radical which can be the same as or different from any other $R^1$ or $R^2$, or each $R^1$ and $R^2$ bonded to the same N atom can combine with one another to form a cyclic structure containing the N atom, and $R^1$ and $R^2$ can contain from 1 to 20 carbon atoms, can be straight chain, branched or cyclic, can be saturated, unsaturated or aromatic, can contain skeletal or catenary hetero atoms other than carbon, and can be unsubstituted or substituted with non-interfering moieties.

**Patentansprüche**

1. Zyklische Anhydride von Perfluorsäuren, dadurch gekennzeichnet, daß die Perfluorsäuren ausgewählt sind aus den Omega-Sulfoperfluoraliphatischcarbonsäuren und den Perfluoraliphatischdisulfonsäuren und daß die zyklischen Anhydride die Formel

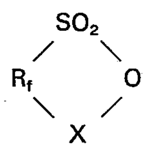

haben, in der $R_f$ ein Perfluoralkylen mit 2 bis 5 Kettenkohlenstoffatomen oder ein Perfluorcycloalkylen mit 4 bis 7 Ringatomen ist, $R_f$ gegebenenfalls mit einer oder mehreren geradkettigen, verzweigten oder zyklischen Perfluoralkylgruppen mit 1 bis 12 Kohlenstoffatomen substituiert ist, $R_f$ insgesamt bis zu 14 Kohlenstoffatome enthält, und X ein Carbonyl- oder Sulfonylradikal ist.

2. Verbindungen nach Anspruch 1, dadurch gekennzeichnet, daß $R_f$ ein Perfluoralkylen mit 2 bis 5 Kettenkohlenstoffatomen ist und $R_f$ gegebenenfalls mit einer oder mehreren geradkettigen oder verzweigten Perfluoralkylgruppen mit 1 bis 4 Kohlenstoffatomen substituiert ist.

3. Verbindungen nach Anspruch 1, dadurch gekennzeichnet, daß $R_f$ $-(CF_2)_m-$ und m gleich 2 bis 4 ist.

4. Verbindung nach Anspruch 3, dadurch gekennzeichnet, daß m gleich 2 ist.

5. Verbindung nach Anspruch 3, dadurch gekennzeichnet, daß m gleich 3 ist.

6. Verbindung nach Anspruch 3, dadurch gekennzeichnet, daß m gleich 4 ist.

7. Verbindungen nach einem der vorhergehenden Ansprüche, dadurch gekennzeichnet, daß X ein Carbonylradikal ist.

8. Verbindungen nach einem der Ansprüche 1 bis 6, dadurch gekennzeichnet, daß X ein Sulfonylradikal ist.

9. Amide, dadurch gekennzeichnet, daß die Amide Derivate der Verbindungen nach Anspruch 1 sind und die Formeln

$$R^1R^2N\overset{O}{\overset{\|}{C}}R_fSO_3^-H_2\overset{+}{N}R^1R^2,$$

$$R^1R^2NSO_2R_fCO_2^-H_2\overset{+}{N}R^1R^2, \text{ oder}$$

$$R^1R^2NSO_2R_fSO_3^-H_2\overset{+}{N}R^1R^2$$

haben, wobei die Verbindungen abgeleitet sind von Aminen, die die Formel $HR^1R^2$ und einen $pk_b$-Wert von 13,2 oder weniger haben, $R_f$ ein Perfluoralkylen mit 2 bis 6 Kettenkohlenstoffatomen oder ein Perfluorcycloalkylen mit 4 bis 7 Ringatomen ist, $R_f$ gegebenenfalls mit einer oder mehreren geradkettigen, verzweigten oder zyklischen Perfluoralkylgruppen mit 1 bis 12 Kohlenstoffatomen substituiert ist, $R_f$ insgesamt bis zu 14 Kohlenstoffatome enthält, $R^1$ und $R^2$ unabhängig voneinander jeweils Wasserstoff oder ein einwertiges organisches Radikal sind, das mit jedem anderen $R^1$ oder $R^2$ gleich oder von ihm verschieden sein kann, oder mit demselben N-Atom verbundene $R^1$ und $R^2$ miteinander derart verbunden sein können, daß sie eine das N-Atom enthaltende, zyklische Struktur bilden, und $R^1$ und $R^2$ 1 bis 20 Kohlenstoffatome enthalten können, geradkettig, verzweigt oder zyklisch sein können, gesättigt, ungesättigt oder aromatisch sein können, Skelett- oder Ketten-Heteroatome außer Kohlenstoff enthalten können, und nichtsubstituiert oder mit nichtstörenden Anteilen substituiert sein können.

**Revendications**

1. Anhydrides cycliques de perfluoroacides, caractérisés en ce que les dits perfluoroacides sont choisis parmi des acides *oméga*-sulfoperfluoroaliphatiquecarboxyliques et des acides perfluoroaliphatique-disulfoniques et les dits anhydrides cycliques sont de formule

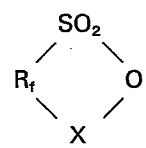

dans laquelle $R_f$ est un perfluoroalkylène comportant 2 à 5 atomes de carbone caténaires ou un perfluorocycloalkylène comportant 4 à 7 sommets, $R_f$ étant éventuellement substitué par un ou plusieurs groupes perfluoroalkyle à chaîne droite, ramifiés ou cycliques de 1 à 12 atomes de carbone, $R_f$ comportant au total jusqu'à 14 atomes de carbone, et X est un radical carbonyle ou sulfonyle.

2. Composé suivant la revendication 1, caractérisé en ce que $R_f$ est un perfluoroalkylène comportant 2 à 5 atomes de carbone caténaires, $R_f$ étant éventuellement substitué par un ou plusieurs groupes perfluoroalkyle à chaîne droite ou ramifiés de 1 à 4 atomes de carbone.

3. Composés suivant la revendication 1, caractérisés en ce que $R_f$ est $-(CF_2)_m-$ et m est 2 à 4.

4. Composé suivant la revendication 3, caractérisé en ce que m est 2.

5. Composé suivant la revendication 3, caractérisé en ce que m est 3.

6. Composé suivant la revendication 3, caractérisé en ce que m est 4.

7. Composés suivant l'une quelconques des revendications précédentes, caractérisés en ce que X est un radical carbonyle.

8. Composés suivant l'une quelconque des revendications 1 à 6, caractérisés en ce que X est un radical sulfonyle.

9. Amides, caractérisés en ce que les dits amides sont dérivés des composés suivant la revendication 1 et sont de formules:

$$R^1R^2N\overset{O}{\overset{\|}{C}}R_fSO_3^-H_2\overset{+}{N}R^1R^2,$$

$$R^1R^2NSO_2R_fCO_2^-H_2\overset{+}{N}R^1R^2, \text{ ou}$$

$$R^1R^2NSO_2R_fSO_3^-H_2\overset{+}{N}R^1R^2$$

dans lesquelles les composés sont dérivés d'amines de formules $HR^1R^2$ ayant un $pk_B$ de 13,2 ou moins, $R_f$ est un perfluoroalkylène comportant 2 à 5 atomes de carbone caténaires ou un perfluorocycloalkylène comportant 4 à 7 sommets, $R_f$ étant éventuellement substitué par un ou plusieurs groupes perfluoroalkyle à chaîne droite, ramifiés ou cycliques de 1 à 12 atomes de carbone, $R_f$ ayant au total jusqu'à 14 atomes de carbone, et chaque $R^1$ et $R^2$ est indépendamment l'hydrogène ou un radical organique monovalent qui peut être le même ou être différent de tout autre $R^1$ ou $R^2$, ou bien chaque $R^1$ et $R^2$ lié au même atome N peut se

combiner avec un autre pour constituer une structure cyclique contenant l'atome N, et $R^1$ et $R^2$ peuvent contenir de 1 à 20 atomes de carbone ils peuvent être à chaîne droite, ramifiés ou cycliques, ils peuvent être saturés, insaturés ou aromatiques, ils peuvent contenir des hétéroatomes de squelette ou caténaires autres que le carbone et ils peuvent être non substitués ou substitués par des groupes n'interférant pas.